# NEW EUROPEAN PATENT SPECIFICATION

(11) **EP 2 790 699 B2**
(45) Date of publication and mention of the opposition decision: **01.01.2020**
(45) Mention of the grant of the patent: 05.04.2017
(21) Application number: 12801538.5
(22) Date of filing: 10.12.2012
(51) Int. Cl.: A61K 31/437, A61K 31/4745, A61K 31/506, A61K 9/14, A61K 9/20, A61K 47/32, A61K 47/38, A61P 35/00

(54) **PHARMACEUTICAL COMPOSITION WITH IMPROVED BIOAVAILABILITY FOR HIGH MELTING HYDROPHOBIC COMPOUND**
PHARMAZEUTISCHE ZUSAMMENSETZUNG MIT VERBESSERTER BIOVERFÜGBARKEIT FÜR EINE HOCHSCHMELZENDE WASSERABWEISENDE VERBINDUNG
COMPOSITION PHARMACEUTIQUE À BIODISPONIBILITÉ AMÉLIORÉE, DESTINÉE À UN COMPOSÉ HYDROPHOBE À POINT DE FUSION ÉLEVÉ

(30) Priority: 13.12.2011 US 201161569863 P
(43) Date of publication of application: 22.10.2014
(73) Proprietor: F. Hoffmann-La Roche AG, 4070 Basel (CH)
(72) Inventor: ALBANO, Antonio, A., Clifton, NJ 07013 (US); DESAI, Dipen, Whippany, NJ 07981 (US); DINUNZIO, James, Cranford, NJ 07016 (US); GO, Zenaida, Clifton, NJ 07013 (US); IYER, Raman, Mahadevan, Piscataway, NJ 08854 (US); SANDHU, Harpreet, K., West Orange, NJ 07052 (US); SHAH, Navnit, Hargovindas, Clifton, NJ 07012 (US)
(74) Representative: Neuhaus, Christian Michel
(86) International application number: PCT/EP2012/074884
(87) International publication number: WO 2013/087546

(56) References cited:
- WO-A1-97/15291
- WO-A1-2010/104945
- WO-A2-2004/069138
- WO-A2-2009/108077
- WO-A2-2010/114928
- TAP WILLIAM D ET AL: "Pharmacodynamic Characterization of the Efficacy Signals Due to Selective BRAF Inhibition with PLX4032 in Malignant Melanoma", NEOPLASIA, NEOPLASIA PRESS, ANN ARBOR, MI, US, vol. 12, no. 8, 1 August 2010 (2010-08-01) , pages 637-649, XP002633497, ISSN: 1522-8002, DOI: 10.1593/NEO.10414

## Description

### Summary of the Invention

The present invention relates to a pharmaceutical composition comprising a solid dispersion of a drug, wherein the drug is molecularly dispersed in said polymer. In the composition, the drug is in substantially amorphous form. WO2010/114928 discloses solid dispersion compositions comprising compound I of the present invention, and different polymers. WO2004/069138 discloses solid dispersion compositions comprising, among other polymers, PVP and compounds which are different from compounds I and II according to the present invention.

The present invention relates to a pharmaceutical composition comprising a solid dispersion comprising a compound of formula (I), 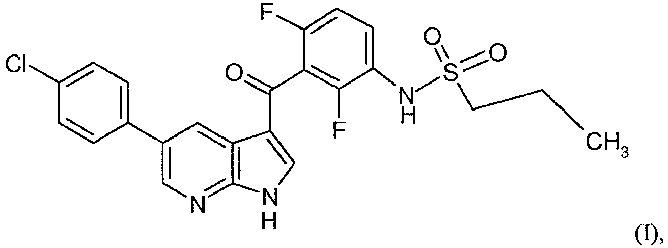 or a compound according to formula (II), 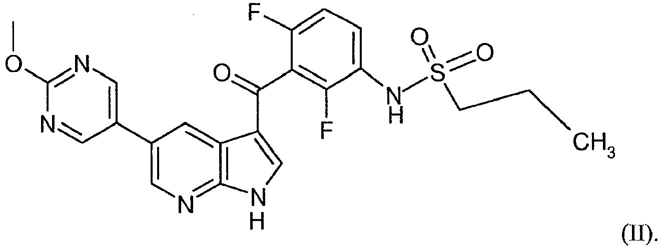 a polymer that is polyvinylpyrrolidone (PVP) or copovidone, and, optionally, a surfactant and/or HPMC-AS.

The present invention also relates to the present compositions for use in a method for treating or ameliorating cancer comprising administering to a subject in need of such treatment a therapeutically effective amount of a composition of the present invention.

### Brief Description of the Figures

Fig. 1: 2-stage non-sink dissolution test results for Formulations 48A, 48B and 48C.
Fig. 2: X-ray diffraction patterns for Formulation 49A and 49C.

### Detailed Description of the Invention

The present invention provides a pharmaceutical composition comprising a solid dispersion comprising a Drug (as defined below) and a polymer as defined in the claims.

As used herein, the term "substantially in amorphous form" means that greater than 50%, or greater than 55%, or greater than 60%, or greater than 65%, or greater than 70%, or greater than 75%, or greater than 80%, or greater than 85%, or greater than 90%, or greater than 95% of the Drug is present in amorphous form.

As used herein, the term "solid dispersion" means any solid composition having at least two components, for example a Drug and a polymer, wherein said drug is molecularly dispersed in said polymer.

As used herein, the term "molecularly dispersed" refers to the random distribution of a Drug with a polymer.

As used herein, the term "solid molecular complex" refers to a solid dispersion that includes a Drug molecularly dispersed within a matrix formed by a polymer (hereafter, a "polymer matrix").

As used herein, the term "immobilized", with reference to the immobilization of a Drug within a polymer matrix, means that the molecules of a Drug interact with the molecules of the polymer in such a way that the molecules of the Drug are held in the aforementioned matrix and prevented from crystal nucleation due to lack of mobility. For example, the polymer may prevent intramolecular hydrogen bonding or weak dispersion forces between two or more Drug molecules.

As used herein, "Drug" refers to either Compound I or Compound II (both defined below). Both Compound I and Compound II are Raf kinase inhibitors. As such, they are useful in treating or ameliorating cancer.

"Compound I", as used herein, refers to propane-1-sulfonic acid {3-[5-(4-chloro-phenyl)-1H-pyrrolo[2,3-b] pyridine-3-carbonyl]-2,4-difluoro-phenyl}-amide. This drug has the following structure.

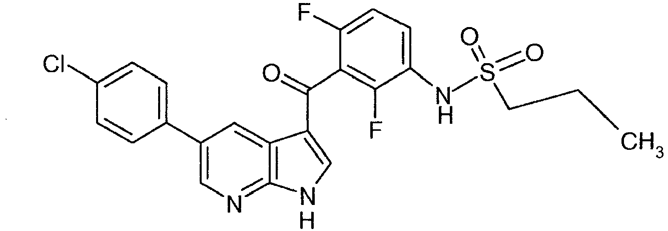

"Compound II", as used herein, refers to propane-1-sulfonic acid {2,4-difluoro-3-[5-(2-methoxy-pyrimidin-5-yl)-1H-pyrrolo[2,3-b]pyridine-3-carbonyl]-phenyl-amide. This drug has the following structure.

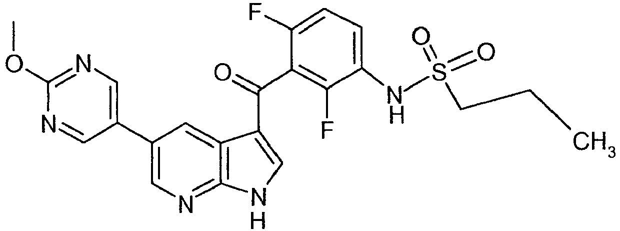

Applicants have found that choice of polymer has a significant effect on AUC and Cₘₐₓ achieved *in vivo* (see Example 8).

In an embodiment, the polymer is polyvinylpyrrolidone (PVP) or copovidone. In a particular embodiment, the polymer is PVP. In another particular embodiment, the polymer is copovidone.

Copovidone (available from BASF and ISP) is a hydrophilic copolymer of 1-vinyl-2-pyrrolidone and vinyl acetate in the mass proportion of 6:4. Copovidone is capable of forming a stable solid dispersion with the Drug which retains the Drug in amorphous form for up to eight hours in the physiological relevant fluid, thus improving its bioavailability upon administration. In addition to the above, copovidone is a non-ionic polymer that has pH independent solubility in the physiological pH range (1.5- 7.5). As a result, a solid dispersion formed using copovidone is capable of releasing the Drug throughout the GI tract, thus allowing for improved absorption of the Drug.

In an embodiment, the Drug is molecularly dispersed in the aforementioned polymer.

In an embodiment, the solid dispersion is a solid molecular complex of Compound I or Compound II and said polymer.

In an embodiment, the Drug is immobilized within a matrix formed by said polymer.

In an embodiment, the composition comprises a solid dispersion wherein the Drug is present in an amount of from about 1% to about 50%, from about 1% to about 40%, or from about 1% to about 30% by weight of the solid dispersion.

In an embodiment, the solid dispersion has a single glass transition temperature higher than about 50°C, preferably above 100 °C.

In an embodiment, the composition comprises a solid dispersion comprising a polymer wherein the polymer is present in an amount of from about 50% to about 98.8%, from about 60% to about 98.8%, or from about 70% to about 98.8% by weight of the solid dispersion.

In an embodiment, the solid dispersion is prepared using a hot melt extrusion process (see, e.g., Ghebre-Sellassie, I. and C. Martin, Pharmaceutical Extrusion Technology, Marcel Dekker, 2003). In such a process, the components of the solid dispersion are blended and extruded at high temperature.

In an embodiment, the composition comprises Compound I molecularly dispersed in copovidone.

In an embodiment, the solid dispersion is a solid molecular complex of Compound I and copovidone.

In an embodiment, Compound I is immobilized within a matrix formed by copovidone.

In an embodiment, the composition comprises a solid dispersion wherein Compound I is present in an amount of from about 1% to about 40% by weight of the solid dispersion and copovidone is present in an amount of from about 60% to about 98.8% by weight of the solid dispersion.

In an embodiment, the composition comprises a solid dispersion wherein Compound I is present in an amount of from about 1% to about 40% by weight of the solid dispersion and copovidone is present in an amount of from about 60% to about 98.8% by weight of the solid dispersion.

In an embodiment, the solid dispersion comprising Compound I and copovidone is prepared using a hot melt extrusion process (see, e.g., Ghebre-Sellassie, I. and C. Martin, Pharmaceutical Extrusion Technology, Marcel Dekker, 2003).

In an embodiment, the composition comprises Compound II molecularly dispersed in copovidone.

In an embodiment, the solid dispersion is a solid molecular complex of Compound II and copovidone.

In an embodiment, Compound II is immobilized within a matrix formed by said copovidone.

In an embodiment, the composition comprises a solid dispersion wherein Compound II is present in an amount of from about 1% to about 50% by weight of the solid dispersion and copovidone is present in an amount of from about 50% to about 98.8% by weight of the solid dispersion.

In an embodiment, the solid dispersion comprising Compound II and copovidone is prepared using a hot melt extrusion process (see, e.g., Ghebre-Sellassie, I. and C. Martin, Pharmaceutical Extrusion Technology, Marcel Dekker, 2003).

In an embodiment of the present invention, the composition further comprises a flow enhancer. In a particular embodiment, the flow enhancer is colloidal silica (also designated herein as colloidal silicon dioxide). The flow enhancer may, for example, be present in the composition in an amount of up to about 5% by weight of the composition, or up to about 3% by weight of the composition. Applicants have found that compositions comprising colloidal silicon dioxide exhibit improved stability and improved AUC and Cₘₐₓ as compared with the composition that did not contain colloidal silicon dioxide (see Example 6).

Melt extrusion formulations exhibit the advantages of good bioavailability and solid state stability. In addition, there are manufacturing advantages to using melt extrusion formulations. It is desirable to develop melt extrusion formulations that also have the advantages of lower dose to achieve sufficient therapeutic effect, low bulk density, high surface area, enhanced drug loading with lower polymer loading, good solubility and excellent physico-chemical properties.

For patient compliance, development of a higher strength dosage form such as a tablet is desirable. Solid dispersion formulations known in the art require a high usage of polymer which may impart undesirable binder effects on tablets, thus slowing tablet disintegration. While disintegrants may be added, the addition of additional excipients may have a negative effect on tablet compaction. It is advantageous to develop other solid dispersion formulation tablets with fast disintegration and good tablet compaction.

Applicants have found that, in embodiments comprising Compound I as the Drug, the addition of a surfactant as a component of certain solid dispersion allows for improved dissolution of Compound I from a solid dispersion. Accordingly, an embodiment of the present invention is a composition comprising a solid dispersion which comprises Compound I, a polymer that is PVP or copovidone, and a surfactant. In an embodiment of the present invention, the surfactant is selected from the group consisting of sodium lauryl sulfate (SLS), glycerol monostearate, dioctyl sodium succinate (DOSS), and mixtures thereof. In an embodiment, the surfactant is SLS. In another embodiment, the surfactant is glycerol monostearate. In yet another embodiment, the surfactant is DOSS. In certain embodiments, the surfactant is present in an amount of up to about 10% by weight of the solid dispersion, or up to about 5% by weight of the solid dispersion, or from about 1% to about 2% by weight of the solid dispersion. In a particular embodiment, the composition comprises a solid dispersion which comprises Compound I, copovidone and DOSS. In a more particular embodiment, DOSS is present in an amount of from about 1% to about 2% by weight of the solid dispersion.

Applicants have found that, in embodiments of the present invention wherein the Drug is Compound II and the polymer is copovidone, the addition of hydroxypropyl methylcellulose - acetate succinate (HPMC-AS) in the solid dispersion allows for improved disintegrating properties for the resulting dosage form. HPMC-AS of various grades may be used, including HPMC-AS, LF; HPMC-AS, M; HPMC-AS, HF; and HPMC-AS, HG. An embodiment of the present invention is a composition comprising a solid dispersion which comprises Compound II, copovidone and HPMC-AS. In another embodiment, the solid dispersion comprises Compound II, copovidone and HPMC-AS, LF. In another embodiment, the solid dispersion comprisies Compound II, copovidone and HPMC-AS, HF. In yet another embodiment, the solid dispersion comprises Compounnd II, copovidone and HPMC-AS, HG. In such embodiments, applicants have found that the ratio of the copovidone to HPMC-AS used in the solid dispersion is of critical importance. In an embodiment, the ratio is from about 15:85 to about 50:50. In another embodiment, the ratio is from about 15:85 to about 40:60. In a particular embodiment, the ratio is about 35:65. In another particular embodiment, the ratio is about 20:80.

In an embodiment, the present invention relates to a pharmaceutical composition comprising a solid dispersion comprising a Drug, a polymer that is polyvinylpyrrolidone (PVP) or copovidone, and, optionally, a surfactant and/or HPMC-AS.

In addition to the above, the present invention contemplates to use of additional components in the present composition. Plasticizers, for example PEG-400 and poloxamer (which also serves as a surfactant), may be used. In addition, disintegrants, for example sodium starch glycolate, Polypasdone XL, and croscarmellose sodium may be used. Further lubricants such as magnesium stearate may be used.

Thus, the present invention also relates to the compositions disclosed herein for use as medicaments, in particular as medicaments for the treatment of cancer, more specifically melanoma.

### Examples

### Example 1

This example describes a formulation of the present invention comprising Compound I. The contents of the formulation were as follows.

| | Wt. % |
|---|---|
| Compound I | 21.5 |
| PVP (Povidone K-90) | 51.6 |
| PEG-400 | 12.9 |
| Poloxamer | 10 |
| Sodium Starch Glycolate | 3 |
| Colloidal Silicon Dioxide (Aerosil 200) | 1 |

The formulation was prepared using the HME process (Ghebre-Sellassie, I. and C. Martin, Pharmaceutical Extrusion Technology, Marcel Dekker, 2003). Compound I, PVP and PEG 400 were mixed and the blend was extruded at 160 ° C. The resulting extrudates were milled by hand. Poloxamer, sodium starch glycolate and colloidal silicon dioxide were added externally to the milled extrudate and blended together to achieve a homogeneous blend.

The blend was filled into hard gelatin capsules.

### Example 2

For comparison, a formulation containing Compound I in stable crystalline form was prepared.

**Stable Crystalline Formulation**

| | Wt. % |
|---|---|
| Compound I | 54.5 |
| ProSolv^{®} (JRS Pharma) | 33 |
| Poloxamer | 10 |
| Sodium Starch Glycolate | 1 |
| Magnesium Stearate | 1 |
| Colloidal Silicon Dioxide (Aerosil 200) | 0.5 |

This formulation was prepared by a dry blending method (Lachman et al., The Theory and Practice of Industrial Pharmacy, Lea & Febiger, 1986). All the components were blended for a suitable time and the resulting dry blend was filled into hard gelatin capsules.

### Example 3

Also for comparison, a formulation containing Compound I in stable crystalline form dissolved in a lipid-based vehicle (the Lipid Formulation) was also prepared.

**Lipid Formulation**

| | | Wt. % |
|---|---|---|
| Compound I | | 10 |
| Labrosol^{®} (Gattefosse) | | 46.8 |
| Gelucire^{®} (Gattefosse) | | 21.6 |
| Vitamin E Tocopherol | Glycol Succinate (Vitamin E - TPGS) | 21.6 |

This formulation was prepared by dispersing Compound I with Labrosol^{®} (Gattefosse), Gelucire^{®} (Gattefosse) and Vitamin E-TPGS in a mortar and pestle. The resulting lipid suspension was then filled into hard gelatin capsules.

### Example 4

A single dose oral PK study using the formulations of Examples 2 and 3 and the solid dispersion formulation of Example 1 was conducted in Female Beagle Dogs using cross over design. All the formulations were dosed at 50 mg/ kg dose level.

Applicants have found that, when Compound I was administered as a solid dispersion (the Example 1 formulation), it exhibited significantly higher bioavailability compared to when Compound I was administered in either the formulations of Examples 2 or 3 wherein Compound I was in crystalline form.

**Table 1: Comparison of Dog PK data - Solid Dispersion vs. Crystalline**

| Formulation | Form of Compound I | AUC/dose | Cₘₐₓ/dose |
|---|---|---|---|
| | | (ng.h/mL) | (ng/mL) |
| Example 2 Formulation | Crystalline | 8-10 | 0.6-1 |
| Example 3 Formulation | Crystalline | 20-24 | 4.5-5.2 |
| Example 1 Formulation | Amorphous | 535-560 | 90-115 |

### Example 5

The miscibility of Compound I in various polymers at constant temperature was analyzed.

Compound I and polymer were mixed to produce a blend that was 10% by weight Compound I and 90% by weight polymer. The homogeneous blend was extruded using a Haake^{®} MiniLab bench-top extruder. The feed rate was constant between 1- 2 g/ min and screw speed was set at 100 RPM. The blends were extruded at two different temperatures: 160 and 200 °C respectively. The extrudates were classified as miscible, partially immiscible, immiscible as per PXRD patterns and visual observations.

Applicants have found that Compound I has higher solubility/ miscibility in copovidone compared to other polymers when melt extruded at 160°C (Table 2).

**Table 2: Miscibility studies for Compound I**

| Polymer with 10% Compound I | Miscibility @ 160 °C | Miscibility @ 200 °C |
|---|---|---|
| Povidone K 30 | Partially immiscible | Miscible |
| Copovidone | Miscible | Miscible |
| Povidone K 90 | Partially immiscible | Miscible |
| Polyvinyl acetate phthalates | Partially immiscible | Polymer degradation |
| Eudragit E 100 | Immiscible | Immiscible |
| HypromellosePartially | Immiscible | Partially immiscible |
| Hypromellose-ASPartially | Immiscible | Polymer degradation |
| Poloxamer | Immiscible | Polymer degradation |

### Example 6

Two formulations, one without and one with colloidal silicon dioxide (Examples 6a and 6b, respectively) were produced as follows.

| | 6a | 6b |
|---|---|---|
| | Wt. % | Wt. % |
| Compound I | 25 | 24 |
| Povidone | 60 | 59 |
| Glyceryl Monostearate | 15 | 15 |
| Aerosil^{®} 200 (colloidal silicon dioxide) | 0 | 2 |

The formulations were processed using Leistriz^{®} Micro 18 lab scale extruder at a constant feed rate of 10-15 g/min, screw speed of 150 rpm and processing temperature in the range of 160- 185 °C. Upon extrusion, the extrudates were milled into fine powder and filled into hard gelatin capsule for testing and evaluation purpose. Both formulations showed glass transition temperature in the range of 110 -120 °C and amorphous PXRD pattern. Both formulations provided similar *in vitro* release profile.

The formulation containing colloidal silicon dioxide was found to be stable for up to 4 hours under normal conditions and also had improved AUC and Cₘₐₓ as compared with the formulation that did not contain colloidal silicon dioxide (see Table 3).

**Table 3: AUC and Cₘₐₓ for Formulations 6a and b**

| | 6a | 6b |
|---|---|---|
| Motor load % | 95-100 | 95-100 |
| Cₘₐₓ/Dose (ng/ml/mg/kg) | 135-200 | 342-370 |
| AUC/Dose (ng*Hours/mL/mg/kg) | 700-2000 | 1500-3600 |

### Example 7

The following evaluation showed that the addition of glyceryl monostearate improved the processability of the formulation (Table 5).

**Table 5: Solid dispersion formulations with or without glyceryl monostearate**

| Example | 7a | 7b | 7c |
|---|---|---|---|
| | | % (w/w) | |
| Compound I | 10 | 10 | 10 |
| Povidone | | | 85 |
| Copovidone | 85 | 90 | |
| Glyceryl Monostearate | 5 | | 5 |
| Processibility ( % motor load) | 50-70 | 90-95 | 40-50 |

### Example 8

Applicants have also found that choice of surfactant and polymer also have significant effect on AUC and Cₘₐₓ. The solid dispersion formulation below containing copovidone and sodium lauryl sulfate provided higher AUC and Cₘₐₓ compared to the solid dispersion formulation containing povidone and glycerol monostearate (see Table 6).

**Table 6: AUC and Cₘₐₓ for Formulations 8a, b and c (Ingredients in %(w/w)).**

| Example | 8a | 8b | 8c |
|---|---|---|---|
| Compound I | 25 | 20 | 20 |
| Povidone | 58 | | |
| Copovidone | | 74 | 78 |
| Glyceryl Monostearate | 15 | 5 | |
| Sodium Lauryl Sulfate | | | 1 |
| Colloidal Silicon (Aerosil 200) | 2 | 1 | 1 |
| Total (%w/w) | 100 | 100 | 100 |
| Cₘₐₓ/Dose (ng/ml/mg/kg) | 342-370 | 500-850 | 600-1050 |
| AUC/Dose (ng*Hours/mL/mg/kg) | 1500-3600 | 2780-4780 | 3540-7560 |

### Examples 9 - 11

Compared to solubilizers such as SLS that also provided higher bioavailability from melt extrudates, tablets containing DOSS provided a better *in vitro* release, suggesting DOSS surprisingly functions as release modifier.

**Table 7: Formulations 9, 10 and 11 (Ingredients in mg/tablet) and dissolution**

| Examples | 9 | 10 | 11 |
|---|---|---|---|
| Compound I | 200 | 200 | 200 |
| Copovidone | 584 | 584 | 576 |
| Colloidal Silicon Dioxide | 8 | 8 | 8 |
| Sodium Lauryl Sulfate | 8 | | |
| Dioctyl Sodium Sulfosucccinate (DOSS) | | 8 | 16 |
| Tablet weight | 800 | 800 | 800 |
| Extrusion temperature (°C) | 160- 185 | 160- 185 | 160- 185 |
| Feed rate ( g/ min) | 10- 20 | 10-20 | 10-20 |
| Screw speed (RPM) | 150-200 | 150-200 | 150-200 |
| PXRD pattern | Amorphous | Amorphous | Amorphous |
| Dissolution ( D60 min) | ∼10% | ∼50% | ∼50% |
| Dissolution ( D 180) | ∼20% | 100% | 100% |
| Compression - hardness(25 kN) | ∼120 N | ∼145N | ∼140 N |

### Examples 12 to 18

The method of addition of solubilizers in the solid dispersion formulation has significant effect on dissolution rate and drug recovery. Intragranular addition of docusate sodium 85% (Dioctyl sodium sulfosuccinate containing 15% sodium benzoate) provided higher dissolution rate and recovery.

**Table 8**

| **Ingredient [%w/w]** | | | | | | | |
|---|---|---|---|---|---|---|---|
| | *Intragranular Excipients* | | | | | | |
| Examples | 12 | 13 | 14 | 15 | 16 | 17 | 18 |
| Compound I | 20 | 25 | 15 | 27 | 20 | 20 | 20 |
| Copovidone | 76.5 | 71.9 | 81.9 | 70 | 75.4 | 75 | 75.5 |
| Docusate sodium 85% | 0.1 | 0.4 | 0.1 | 0.4 | 1 | 2 | 0.5 |
| Colloidal Silicon Dioxide | 0.1 | 0.1 | 0.1 | 0.1 | 0.2 | 0.3 | 0.3 |

| | *Extragranular Excipients* | | | | | | |
|---|---|---|---|---|---|---|---|
| Colloidal Silicon Dioxide | 0.1 | 0.1 | 0.2 | 0.1 | 0.2 | 0.1 | 0.2 |
| Glyceryl behenate | 0.8 | 0.5 | 0.2 | 0.5 | 0.8 | 0.2 | 0.5 |
| Opadry II | 2.4 | 2 | 2.5 | 1.9 | 2.4 | 2.4 | 3 |

### Example 19

This example describes a formulation of the present invention comprising Compound I.

| | [mg/tablet] |
|---|---|
| **Intragranular Excipients** | |
| Compound I | 240.0 |
| Copovidone ¹ | 940.0 |
| Docusate sodium 85%^{1,2} | 10.0 |
| Colloidal Silicon Dioxide ¹ | 10.0 |

| **Extragranular Excipients** | |
|---|---|
| Colloidal Silicon Dioxide | 2.0 |
| Glyceryl behenate | 8.0 |
| | |
| **Kernal weight** | 1210.0 |

| **Coating composition** | |
|---|---|
| Opadry II Pink ³ | 30.0 |
| **Total tablet Weight** | 1240.0 |
| 1. These four ingredients were the components of the powder mixture which was processed (extruded) through the Leistritz extruder. | |
| 2. Dioctyl sodium sulfosuccinate containing 15% sodium benzoate | |
| 3. Complete coating system | |

Compound I, copovidone, docusate sodium 85% and colloidal silicon dioxide were blended and extruded using Leistriz Micro 18 lab scale extruder. The feed rate was constant between 10-15 g/ min and screw speed was set at 150 RPM. The processing temperature was set in between 160- 185 °C. The extrudates were milled and external components- colloidal silicon dioxide and glycerol behenate- were added and blended for 15 min using suitable powder blender. The blend was compressed into tablet with hardness in the rage of 110 to 180 N hardness. The tablets were coated with Opadry II pink complete coating system.

### Example 20

This example describes a formulation of the present invention comprising Compound II. The contents of the formulation were as follows.

| | Wt. % |
|---|---|
| Compound II | 15.1 |
| Copovidone (Kollidon 64) | 20.8 |
| HPMC-AS, LF | 38.8 |
| Colloidal Silicon Dioxide (Aerosil 200) | 1.8 |
| Microcrystalline cellulose (Avicel PH 102) | 15.0 |
| Polyplasdone XL | 5.0 |
| Croscarmellose sodium (AcDiSol) | 3.0 |
| Magnesium Stearate | 0.5 |

The formulation was prepared using the HME process (Ghebre-Sellassie, I. and C. Martin, Pharmaceutical Extrusion Technology, Marcel Dekker, 2003). Compound II, copovidone and HPMC-AS were mixed and the blend was extruded at 160 ° C. The resulting extrudates were milled by hand. Colloidal sodium dioxide, microcrystalline cellulose, Polyplasdone XL, croscarmellose sodium, and magnesium stearate were added externally to the milled extrudate and blended together to achieve a homogeneous blend.

### Examples 21 to 32a

The following are additional compositions comprising Compound II wherein Compound II is contained in amorphous form. The amounts are expressed in wt% of the composition.

**Table 9**

| **Example** | **Compound II** | **Copovidone** | **HPMC-AS** | **Covpovidone/ HPMC-AS ratio** | **Additional Components** |
|---|---|---|---|---|---|
| | | | | | |
| 21 | 25 | 74 | none | 100/0 | 1% SLS |
| 22 | 25 | 55.5 | 18.5 | 75/25 | 1% SLS |
| 23 | 20 | 40 | 40 | 50/50 | no SLS |
| 24 | 20 | 39.5 | 39.5 | 50/50 | 1%SLS |
| 25 | 20 | 39.9 | 39.9 | 50/50 | 0.2% SLS |
| 26 | 20 | 70 | none | 100/0 | 10% Cremophor |
| 27 | 20 | 79 | none | 100/0 | 1% DOSS |
| 28 | 20 | 37 | 37 | 50/50 | 5%Cremophor, 1% DOSS |
| 29 | 20 | 39 | 39 | 50/50 | 1% DOSS |
| 30 | 20 | 31 | 47 | 40/60 | 1% DOSS, 1% silica |
| 31 | 20 | 37 | 37 | 50/50 | 5% Span, 1 % silica |
| 32a | 10 | none | 87 | 0/100 | 2% DOSS, 1% silica |

### Examples 32b to 47

The following are additional compositions comprising Compound II wherein Compound II is contained in amorphous form. The amounts are expressed in wt % of the composition. With the exception of Example 43, each composition was loaded into tablets which were 75.5% by weight of tablet was the composition. The tablets formed using the composition of Examples 36 and 41 showed no disintegration. The tablets formed using the compositions of example 32b to 35, 37 to 40, 42, and 44 to 47 showed disintegration. For Example 43, tablets containing the composition at 60% to 75% by weight showed no disintegration.

**Table 10**

| **Example** | **%drug** | **% Copovidone** | **%HPMC-AS** | **Copovidone/ HPMC-AS ratio** |
|---|---|---|---|---|
| 32b | 20 | 31.6 | 47.4 | 40/60 |
| 33 | 20 | 23.7 | 55.3 | 30/70 |
| 34 | 20 | 27.6 | 51.4 | 35/65 |
| 35 | 20 | 31.6 | 47.4 | 40/60 |
| 36 | 20 | 51.4 | 27.6 | 65/35 |
| 37 | 15 | 29.4 | 54.6 | 35/65 |
| 38 | 20 | 27.6 | 51.4 | 35/65 |
| 39 | 25 | 29.6 | 44.4 | 40/60 |
| 40 | 25 | 37 | 37 | 50/50 |
| 41 | 40 | 59 | none | 100/0 |
| 42 | 30 | 34.5 | 34.5 | 50/50 |
| 43 | 40 | 59 | none | 100/0 |
| 44 | 20 | 39.5 | 39.5 | 50/50 |
| 45 | 25 | 37 | 37 | 50/50 |
| 46 | 25 | 29.6 | 44.4 | 40/60 |
| 47 | 30 | 34.5 | 34.5 | 50/50 |

### Example 48

This example describes formulations of the present invention utilizing different grades of HPMCAS and polymeric ratios prepared by hot melt extrusion. The compositions of the formulations are presented in Table 11. Formulation 48A was prepared by tumble blending of drug and colloidal silicon dioxide, followed by delumping using a rotary impeller mill with a 0.055" screen and final blending with polymeric excipients. Melt extrusion was conducted using a Leistritz 18-mm twin screw co-rotating extruder in a 20:1 configuration with 3 mm die at a processing temperature of 175°C. Formulations 48B and 48C were manufactured by tumble blending drug and polymeric excipients prior to melt extrusion. Melt extrusion was conducted using a Haake Minilab conical twin screw extruder maintained at a temperature of 175°C.

**Table 11 - Compound II Melt Extruded Formulations Expressed as a Percentage of Total Extrudate Amount**

| Material | Formulation 48A | Formulation 48B | Formulation 48C |
|---|---|---|---|
| Compound II | 20.00 | 20.0 | 20.0 |
| Copovidone | 27.65 | 16.0 | 16.0 |
| HPMCAS-LF | 51.35 | -- | -- |
| HPMCAS-MF | -- | 64.0 | -- |
| HPMCAS-HF | -- | -- | 64.0 |
| Colloidal Silicon Dioxide | 1.00 | -- | -- |

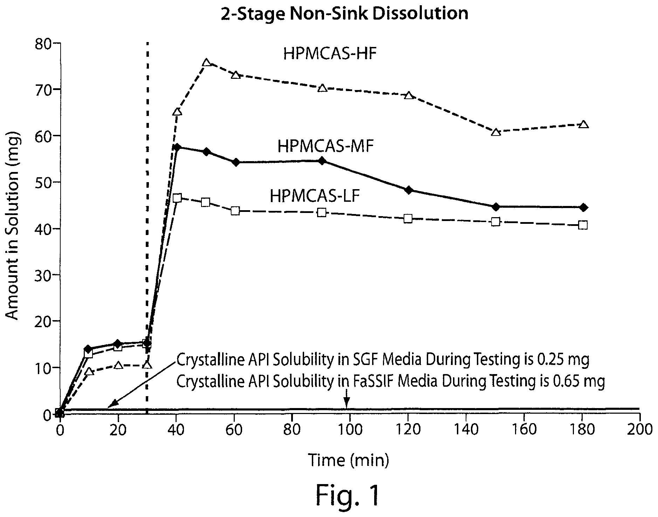

Following extrusion, all dispersions were milled, screened to a fine powder having a size approximately less than 250 microns and tested for dissolution performance under non-sink conditions applying a 2-stage dissolution test. Dissolution profiles for each formulation, tested as powder containing 250 mg equivalent of Compound II, were monitored using a fiber-optic probe and USP apparatus II 6-vessel dissolution assembly implementing a pH change methodology. First stage media was pH 2 simulated gastric fluid without enzyme at a total volume of approximately 500 ml. The second stage media was a biorelevant FaSSIF media at pH 6.5, obtained by adding concentrate to the acidic volume of the first stage to achieve a total volume of approximately 1000 ml. Profiles for each formulation, presented in Figure 1, show greater levels of drug in solution than the crystalline solubilities of Compound II.

Melt extruded solid dispersions of Formulation A and Formulation C were also administered to beagle dogs (n = 6) as a 75 mg/ml total solids oral suspension in a pH 4.0 2.0% hydroxypropyl cellulose vehicle at a dose of 75 mg API/kg. The pharmacokinetic measurements of Compound II are presented in Table 12.

**Table 12 -Pharmacokinetic Measurements of Compound II at 75 mg/kg in Beagle Dogs. Data Presented as Mean Value ± Standard Deviation**

| Metric | Formulation 48A | Formulation 48C |
|---|---|---|
| AUC₀₋₂₄ | 244,000 ± 165,000 | 352,000 ± 258,000 |
| Cₘₐₓ | 24,000 ± 9,100 | 39,200 ± 14,900 |

### Example 49

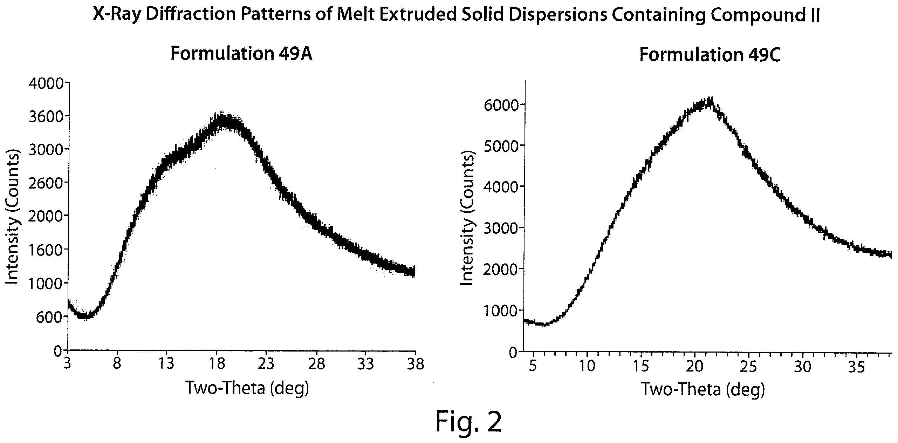

This example describes formulations of the present invention utilizing differing Copovidone:HPMCAS-HF ratios to increase the amount of Compound II contained within the dispersion in a substantially amorphous state when prepared by hot melt extrusion at 175°C. The compositions of each formulation are presented in 13 along with critical product and process attributes. Formulations 49A, 49B, 49C and 49D were manufactured by tumble blending drug and polymeric excipients prior to melt extrusion. Melt extrusion was conducted using a Haake Minilab conical twin screw extruder maintained at a temperature of 175°C and screw speed of 360 rpm. The appearance of a transparent amber glass from the die exit was used to identify amorphous materials which were confirmed by x-ray diffraction testing performed on milled powder samples of solid dispersion. Representative diffraction patterns for Formulation 49A and Formulation 49C are shown in Figure 2.

**Table 13 - Compound II Hot Melt Extruded Formulation, Process and Product Attributes**

| Metric | Formulation 49A | Formulation 49B | Formulation 49C | Formulation 49D |
|---|---|---|---|---|
| FORMULATION | | | | |
| Compound II | 20.00 | 25.0 | 30.0 | 35.0 |
| Copovidone | 16.00 | 15.0 | 35.0 | 32.5 |
| HPMCAS-HF | 64.00 | 60.0 | 35.0 | 32.5 |

| MANUFACTURING | | | | |
|---|---|---|---|---|
| Temp. [°C] | 175 | 175 | 175 | 175 |
| Appearance | Clear Glass | Opaque | Clear Glass | Opaque |
| XRD | Amorphous | Not Tested | Amorphous | Not Tested |

## Claims

1. A pharmaceutical composition comprising a solid dispersion comprising a polymer that is polyvinylpyrrolidone (PVP) or copovidone, a compound according to formula (I), 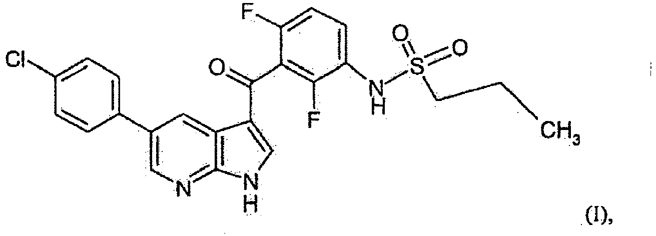 or a compound according to formula (II), 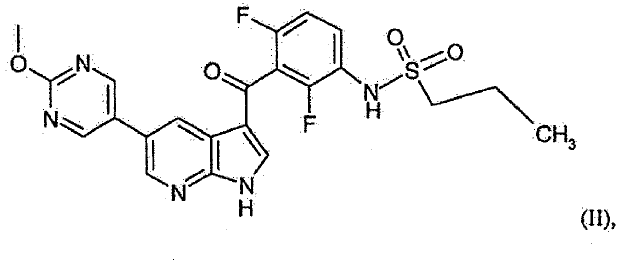 and, optionally, a surfactant and/or hydroxypropyl methylcellulose -acetate succinate, wherein said compound is molecularly dispersed in said polymer.

2. A composition according to claim 1 wherein said polymer is copovidone.

3. A composition according to claim 1 or 2 wherein said solid dispersion is prepared using a hot melt extrusion process.

4. A composition according to any one of claims 1 to 3 further comprising a flow enhancer.

5. A composition according to claim 4 wherein said flow enhancer is colloidal silicone.

6. A composition according to claim 4 or 5 wherein said flow enhancer is present in an amount of up to about 5% by weight of the composition.

7. A composition according to any one of claims 1 to 6 wherein said polymer is copovidone and said solid dispersion comprises a surfactant.

8. A composition according to claim 7 wherein said surfactant is selected from the group consisting of sodium lauryl sulfate (SLS), glycerol monostearate, dioctyl sodium succinate (DOSS), and mixtures thereof.

9. A composition according to claim 7 or 8 wherein said surfactant is dioctyl sodium succinate.

10. A composition according to any one of claims 7 to 9 wherein said surfactant is present in an amount of up to about 10% by weight of said solid dispersion.

11. A composition according to any one of claims 1 to 10 wherein said compound is a compound of formula (I).

12. A composition according to any one of claims 1 to 10 wherein said compound is a compound of formula (II).

13. A composition according to any one of claims 1 to 6 wherein said compound is a compound of formula (II) and said polymer is copovidone and said solid dispersion comprises hydroxypropyl methylcellulose - acetate succinate.

14. A composition according to claim 13 wherein said copovidone and said hydroxypropyl methylcellulose - acetate succinate are present in the solid dispersion in a ratio of from about 15:85 to about 40:60, respectively.

15. A composition according to any one of claims 1 to 14 for use as medicament.

16. A composition according to any one of claims 1 to 14 for use as medicament for the treatment of cancer, in particular melanoma.

## Patentansprüche

1. Pharmazeutische Zusammensetzung, umfassend eine feste Dispersion, umfassend ein Polymer, das Polyvinylpyrrolidon (PVP) oder Copovidon ist, eine Verbindung gemäß Formel (I), 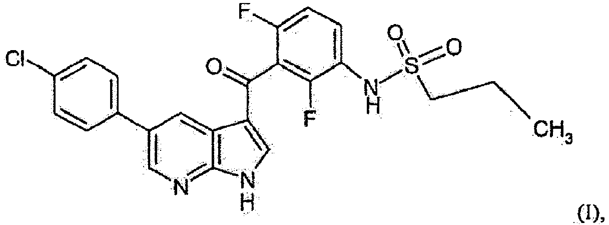 oder eine Verbindung gemäß Formel (II), 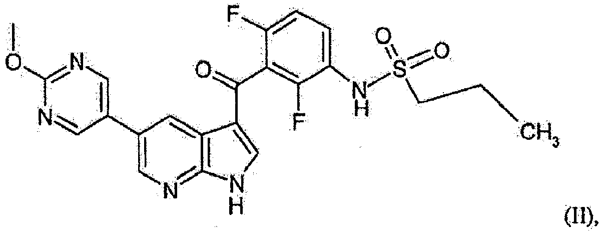 und gegebenenfalls ein oberflächenaktives Mittel und/oder Hydroxypropylmethylcellulose-acetatsuccinat, wobei die Verbindung in dem Polymer molekular dispergiert ist.

2. Zusammensetzung nach Anspruch 1, wobei das Polymer Copovidon ist.

3. Zusammensetzung nach Anspruch 1 oder 2, wobei die feste Dispersion unter Anwendung eines Heißschmelzextrusionsverfahrens hergestellt wird.

4. Zusammensetzung nach einem der Ansprüche 1 bis 3, ferner umfassend einen Fließverbesserer.

5. Zusammensetzung nach Anspruch 4, wobei der Fließverbesserer kolloidales Silikon ist.

6. Zusammensetzung nach Anspruch 4 oder 5, wobei der Fließverbesserer in einer Menge von bis zu etwa 5 Gew.-% der Zusammensetzung vorliegt.

7. Zusammensetzung nach einem der Ansprüche 1 bis 6, wobei das Polymer Copovidon ist und die feste Dispersion ein oberflächenaktives Mittel umfasst.

8. Zusammensetzung nach Anspruch 7, wobei das oberflächenaktive Mittel ausgewählt ist aus der Gruppe, bestehend aus Natriumlaurylsulfat (SLS), Glycerinmonostearat, Dioctylnatriumsuccinat (DOSS) und Gemischen davon.

9. Zusammensetzung nach Anspruch 7 oder 8, wobei das oberflächenaktive Mittel Dioctylnatriumsuccinat ist.

10. Zusammensetzung nach einem der Ansprüche 7 bis 9, wobei das oberflächenaktive Mittel in einer Menge von bis zu etwa 10 Gew.-% der festen Dispersion vorliegt.

11. Zusammensetzung nach einem der Ansprüche 1 bis 10, wobei die Verbindung eine Verbindung der Formel (I) ist.

12. Zusammensetzung nach einem der Ansprüche 1 bis 10, wobei die Verbindung eine Verbindung der Formel (II) ist.

13. Zusammensetzung nach einem der Ansprüche 1 bis 6, wobei die Verbindung eine Verbindung der Formel (II) ist und das Polymer Copovidon ist und die feste Dispersion Hydroxypropylmethylcellulose-acetatsuccinat umfasst.

14. Zusammensetzung nach Anspruch 13, wobei das Copovidon und das Hydroxypropylmethylcellulose-acetatsuccinat in der festen Dispersion jeweils in einem Verhältnis von etwa 15 : 85 bis etwa 40 : 60 vorliegen.

15. Zusammensetzung nach einem der Ansprüche 1 bis 14 zur Verwendung als ein Medikament.

16. Zusammensetzung nach einem der Ansprüche 1 bis 14 zur Verwendung als ein Medikament zur Behandlung von Krebs, insbesondere Melanom.

## Revendications

1. Composition pharmaceutique comprenant une dispersion solide comprenant un polymère qui est la polyvinylpyrrolidone (PVP) ou la copovidone, un composé selon la formule (I), 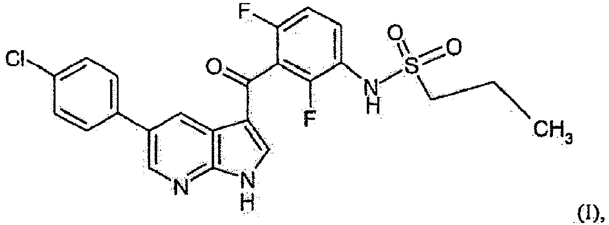 ou un composé selon la formule (II), 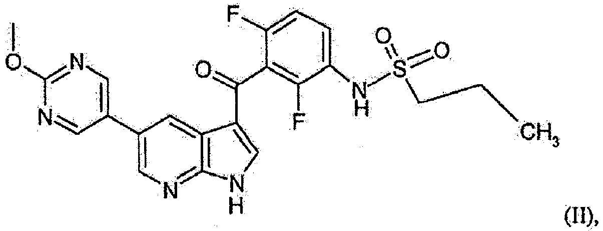 et, éventuellement, un tensioactif et/ou de l'acétate-succinate d'hydroxypropylméthylcellulose, dans laquelle ledit composé est moléculairement dispersé dans ledit polymère.

2. Composition selon la revendication 1 dans laquelle ledit polymère est la copovidone.

3. Composition selon la revendication 1 ou 2 dans laquelle ladite dispersion solide est préparée en utilisant un procédé d'extrusion par fusion à chaud.

4. Composition selon l'une quelconque des revendications 1 à 3 comprenant en outre un agent fluidifiant.

5. Composition selon la revendication 4 dans laquelle ledit agent fluidifiant est une silicone colloïdale.

6. Composition selon la revendication 4 ou 5 dans laquelle ledit agent fluidifiant est présent en une quantité allant jusqu'à environ 5 % en poids de la composition.

7. Composition selon l'une quelconque des revendications 1 à 6 dans laquelle ledit polymère est la copovidone et ladite dispersion solide comprend un tensioactif.

8. Composition selon la revendication 7 dans laquelle ledit tensioactif est choisi dans le groupe constitué par le laurylsulfate de sodium (SLS), le monostéarate de glycérol, le dioctylsuccinate de sodium (DOSS), et des mélanges de ceux-ci.

9. Composition selon la revendication 7 ou 8 dans laquelle ledit tensioactif est le dioctylsuccinate de sodium.

10. Composition selon l'une quelconque des revendications 7 à 9 dans laquelle ledit tensioactif est présent en une quantité allant jusqu'à environ 10 % en poids de ladite dispersion solide.

11. Composition selon l'une quelconque des revendications 1 à 10 dans laquelle ledit composé est un composé de formule (I).

12. Composition selon l'une quelconque des revendications 1 à 10 dans laquelle ledit composé est un composé de formule (II).

13. Composition selon l'une quelconque des revendications 1 à 6 dans laquelle ledit composé est un composé de formule (II) et ledit polymère est la copovidone et ladite dispersion solide comprend de l'acétate-succinate d'hydroxypropylméthylcellulose.

14. Composition selon la revendication 13 dans laquelle ladite copovidone et ledit acétate-succinate d'hydroxypropylméthylcellulose sont respectivement présents dans la dispersion solide selon un rapport d'environ 15:85 à environ 40:60.

15. Composition selon l'une quelconque des revendications 1 à 14 pour une utilisation comme médicament.

16. Composition selon l'une quelconque des revendications 1 à 14 pour une utilisation comme médicament dans le traitement d'un cancer, en particulier d'un mélanome.
